(19) Europäisches Patentamt / European Patent Office / Office européen des brevets

(11) EP 4 684 790 A1

(12) # EUROPEAN PATENT APPLICATION
published in accordance with Art. 153(4) EPC

(43) Date of publication:
**28.01.2026 Bulletin 2026/05**

(21) Application number: **24843538.0**

(22) Date of filing: **19.07.2024**

(51) International Patent Classification (IPC):
***A61K 33/44** (2006.01)* ***A61P 17/14** (2006.01)*
***A61P 43/00** (2006.01)*

(52) Cooperative Patent Classification (CPC):
**A61K 33/44; A61P 17/14; A61P 43/00**

(86) International application number:
**PCT/KR2024/010403**

(87) International publication number:
**WO 2025/018824 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.07.2023 KR 20230094526**
**18.07.2024 KR 20240095224**

(71) Applicants:
- **Qarbonx Inc.**
**Daejeon 34051 (KR)**
- **Korea Advanced Institute of Science and Technology**
**Guseong-dong, Yuseong-gu**
**Daejeon 34141 (KR)**

(72) Inventor: **CHO, Sungjae**
**Daejeon 34185 (KR)**

(74) Representative: **Manitz Finsterwald**
**Patent- und Rechtsanwaltspartnerschaft mbB**
**Martin-Greif-Straße 1**
**80336 München (DE)**

(54) **GRAPHENE OXIDE-BASED COMPOSITION FOR PREVENTING OR ALLEVIATING HAIR LOSS**

(57) The present disclosure relates to a graphene oxide-based a composition for preventing hair loss or promoting hair growth, including a graphene oxide. According to embodiments of the present disclosure, a composition for preventing hair loss or promoting hair growth may exhibit excellent effects in promoting the growth or proliferation of human follicle dermal papilla cells, in enhancing the expression or activity of hair growth-promoting factor proteins in human follicle dermal papilla cells, and in improving the expression or activity of Wnt/β-catenin. Also, the composition shows superior suppression of hair loss-inducing gene expression. Thus, the composition exhibits excellent effects in preventing hair loss or promoting hair growth.

[FIG. 1]

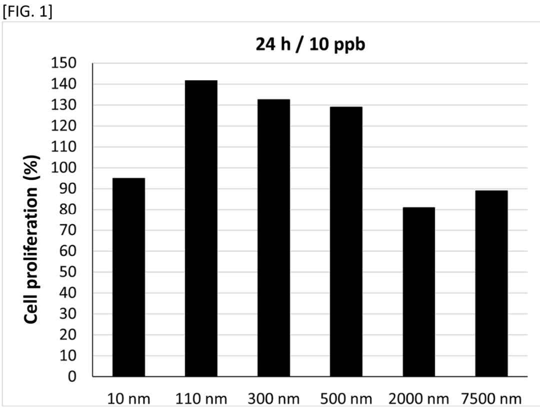




Processed by Luminess, 75001 PARIS (FR)

## Description

### TECHNICAL FIELD

The present disclosure relates to a composition for preventing hair loss or promoting hair growth, including a graphene oxide.

### BACKGROUND

Hair loss, once regarded as a condition primarily affecting middle-aged men, has become a widespread disorder across all age groups and genders, leading to steady growth in the hair loss treatment market. According to 2024 research by UBIST, a pharmaceutical market research firm, the domestic hair loss treatment market grew from about 99.0 billion KRW in 2021 to 102.4 billion KRW in 2023. Further, Research and Markets, a global market research organization, reported that the global hair loss treatment market, valued at about 3.5 billion USD in 2020, is expected to reach about 6.2 billion USD by 2027, with an annual growth rate of 8.4%. In addition to hair loss medication, interest in hair loss prevention shampoos, health functional foods, and medical devices is also gradually increasing.

Androgenetic alopecia is a type of hair loss caused by the overexpression of the male hormone testosterone and accounts for the majority of male hair loss cases. When testosterone binds to 5$\alpha$-reductase in hair follicles, it is reduced to dihydrotestosterone (DHT), a hormone known as the main cause of hair loss. DHT inhibits hair growth in hair follicles and induces hair loss. As of 2024, the active ingredients approved for hair loss treatment in Korea include finasteride, dutasteride, and minoxidil. Finasteride and dutasteride were originally developed to treat benign prostatic hyperplasia. However, at low doses, they have been found to be effective in treating hair loss by inhibiting the overexpression of male hormones. Minoxidil, initially developed to treat hypertension, was additionally approved as a hair loss medication after its side effect of hypertrichosis was identified. When it is applied topically to the scalp, it dilates blood vessels, increases blood flow, and promotes hair growth.

However, finasteride and dutasteride carry the risk of systemic side effects such as decreased libido and erectile dysfunction due to their mechanism of inhibiting male hormones. Further, if a pregnant woman or a woman who may become pregnant comes into contact with the medication, it may adversely affect a male fetus.

[PRIOR ART DOCUMENT]

[Patent literature]

Republic of Korea Registered Patent 10-2506673

### DISCLOSURE OF THE INVENTION

### PROBLEMS TO BE SOLVED BY THE INVENTION

The present disclosure provides a composition for preventing hair loss or promoting hair growth, including a graphene oxide.

However, problems to be solved by the present disclosure are not limited to the above-described problems, and although not described herein, other problems to be solved by the present disclosure can be clearly understood by those skilled in the art from the following descriptions.

### MEANS FOR SOLVING THE PROBLEMS

A first aspect of the present disclosure provides a composition for preventing hair loss or promoting hair growth, including a graphene oxide, wherein an average diameter of the graphene oxide is about 100 nm or more.

A second aspect of the present disclosure provides a method for preventing hair loss or promoting hair growth, including administering to a subject the composition according to the first aspect.

A third aspect of the present disclosure provides a cosmetic composition for preventing hair loss or promoting hair growth, including a graphene oxide, wherein an average diameter of the graphene oxide is about 100 nm or more.

A fourth aspect of the present disclosure provides a quasi-drug composition for preventing hair loss or promoting hair growth, including a graphene oxide, wherein an average diameter of the graphene oxide is about 100 nm or more.

A fifth aspect of the present disclosure provides a pharmaceutical composition for preventing hair loss or promoting hair growth, including a graphene oxide, wherein an average diameter of the graphene oxide is about 100 nm or more.

## EFFECTS OF THE INVENTION

According to embodiments of the present disclosure, a composition for preventing hair loss or promoting hair growth may exhibit excellent effects in promoting the growth or proliferation of human follicle dermal papilla cells, in enhancing the expression or activity of hair growth-promoting factor proteins in human follicle dermal papilla cells, and in improving the expression or activity of Wnt/β-catenin. Also, the composition shows superior suppression of hair loss-inducing gene expression. Thus, the composition exhibits excellent effects in preventing hair loss or promoting hair growth.

In an embodiment of the present disclosure, the composition for preventing hair loss or promoting hair growth contains graphene oxide having an average diameter of about 100 nm or more. Compared to graphene oxide having an average diameter of less than 100 nm, the composition may exhibit superior effects in promoting the growth or proliferation of human follicle dermal papilla cells, in enhancing the expression or activity of hair growth-promoting factor proteins in human follicle dermal papilla cells, and in improving the expression or activity of Wnt/β-catenin. Also, the composition shows superior suppression of hair loss-inducing gene expression. Thus, the composition exhibits excellent effects in preventing hair loss or promoting hair growth.

In an embodiment of the present disclosure, the composition for preventing hair loss or promoting hair growth is administered or applied topically to the treatment site to exhibit effects in preventing hair loss or promoting hair growth. Thus, the composition may prevent side effects associated with conventional hair loss medications (for example, systemic side effects caused by inhibition of sex hormones).

## BRIEF DESCRIPTION OF THE DRAWINGS

**FIG. 1** illustrates the proliferation rate of human follicle dermal papilla cells depending on the size (average diameter) of graphene oxide after 24 hours of culture at a graphene oxide concentration of 10 ppb according to an example of the present disclosure.

**FIG. 2** illustrates the proliferation rate of human follicle dermal papilla cells depending on the size of graphene oxide after 72 hours of culture at a graphene oxide concentration of 10 ppb according to an example of the present disclosure.

**FIG. 3** illustrates the proliferation rate of human follicle dermal papilla cells depending on the concentration of graphene oxide of 110 nm in size after 24 hours of culture according to an example of the present disclosure.

**FIG. 4** shows optical microscopic images of human follicle dermal papilla cells depending on the concentration of graphene oxide of 110 nm in size after 24 hours of culture according to an example of the present disclosure.

**FIG. 5** and **FIG. 6** illustrate the production amounts of hair growth-promoting factor proteins (FGF7 in **FIG. 5** and VEGF in **FIG. 6**) in human follicle dermal papilla cells depending on treatment with graphene oxide of 10 nm or 110 nm in size (concentration of 10 ppb) according to an example of the present disclosure.

**FIG. 7** illustrates the result of evaluation of superoxide dismutase (SOD) enzyme activity in human follicle dermal papilla cells depending on treatment with graphene oxide of 10 nm or 110 nm in size (concentration of 10 ppb) according to an example of the present disclosure.

**FIG. 8A** to **FIG. 8C** illustrate the expression levels of hair loss-inducing genes (AR in **FIG. 8A**, 5αR1 in **FIG. 8B**, and DKK1 in **FIG. 8C**) in human follicle dermal papilla cells depending on treatment with graphene oxide of 10 nm or 110 nm in size according to an example of the present disclosure.

**FIG. 9** illustrates the result of evaluation of Wnt/β-catenin signaling activity in human follicle dermal papilla cells depending on treatment with graphene oxide of 10 nm or 110 nm in size according to an example of the present disclosure.

**FIG. 10A** to **FIG. 10D** illustrate the results of evaluation of mRNA expression of cytokines (HGF in **FIG**. **10A**, IGF-1 in **FIG. 10B**, VEGF in **FIG. 10C**, and FGF7 in **FIG. 10D**) in human follicle dermal papilla cells depending on treatment with graphene oxide of 10 nm or 110 nm in size (100 ppb) according to an example of the present disclosure.

**FIG. 11** and **FIG. 12** illustrate the result of evaluation of hair growth (**FIG. 11**) and corresponding images (**FIG. 12**) depending on treatment with PDDA-functionalized graphene oxide (PDDA-GO) of 10 nm or 110 nm in size according to an example of the present disclosure.

## BEST MODE FOR CARRYING OUT THE INVENTION

Hereinafter, embodiments and examples of the present disclosure will be described in detail with reference to the accompanying drawings so that the present disclosure may be readily implemented by those skilled in the art. However, it is to be noted that the present disclosure is not limited to the embodiments and examples but can be embodied in various

other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element.

Through the whole document, the term "on" that is used to designate a position of one element with respect to another element includes both a case that the one element is adjacent to the other element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Through the whole document, the term "about or approximately" or "substantially" is intended to have meanings close to numerical values or ranges specified with an allowable error and intended to prevent accurate or absolute numerical values disclosed for understanding of the present disclosure from being illegally or unfairly used by any unconscionable third party.

Through the whole document, the term "step of" does not mean "step for".

Through the whole document, the term "combination(s) of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Through the whole document, a phrase in the form "A and/or B" means "A or B, or A and B".

Throughout the whole document, the term "hair loss" refers to a condition in which hair is either absent from an area where it is typically found and/or the number of hairs is reduced compared to a normal state.

Throughout the whole document, the term "diameter" of graphene oxide refers to the length of the longest line passing through the center of a graphene oxide particle, from one edge to the opposite edge.

Throughout the whole document, the term "average diameter" of graphene oxide refers to the mean value of the diameter distribution of the graphene oxide.

Hereinafter, embodiments of the present disclosure are described in detail. However, the present disclosure is not limited thereto.

A first aspect of the present disclosure provides a composition for preventing hair loss or promoting hair growth, including a graphene oxide, wherein an average diameter of the graphene oxide is about 100 nm or more.

In an embodiment of the present disclosure, the average diameter of the graphene oxide may be about 100 nm or more. In an embodiment of the present disclosure, the average diameter of the graphene oxide may be about 2,000 nm or less, about 1,500 nm or less, about 1,000 nm or less, about 900 nm or less, about 800 nm or less, about 700 nm or less, about 600 nm or less, about 500 nm or less, about 450 nm or less, about 400 nm or less, about 350 nm or less, or about 300 nm or less. In an embodiment of the present disclosure, the average diameter of the graphene oxide may be about 100 nm to about 2,000 nm, about 100 nm to about 1,500 nm, about 100 nm to about 1,000 nm, about 100 nm to about 900 nm, about 100 nm to about 800 nm, about 100 nm to about 700 nm, about 100 nm to about 600 nm, about 100 nm to about 500 nm, about 100 nm to about 450 nm, about 100 nm to about 400 nm, about 100 nm to about 350 nm, or about 100 nm to about 300 nm.

In an embodiment of the present disclosure, the average diameter of the graphene oxide may be about 100 nm to about 500 nm. In an embodiment of the present disclosure, the average diameter of the graphene oxide may be about 100 nm to about 300 nm.

In an embodiment of the present disclosure, an average thickness of the graphene oxide may be about 100 nm to about 2,000 nm, about 100 nm to about 1,500 nm, about 100 nm to about 1,000 nm, about 100 nm to about 900 nm, about 100 nm to about 800 nm, about 100 nm to about 700 nm, about 100 nm to about 600 nm, about 100 nm to about 500 nm, about 100 nm to about 450 nm, about 100 nm to about 400 nm, about 100 nm to about 350 nm, or about 100 nm to about 300 nm.

In an embodiment of the present disclosure, the graphene oxide may be functionalized with a quaternary ammonium salt.

In an embodiment of the present disclosure, the graphene oxide may be functionalized with a quaternary ammonium salt represented by the following Chemical Formula 1:

[Chemical Formula 1]

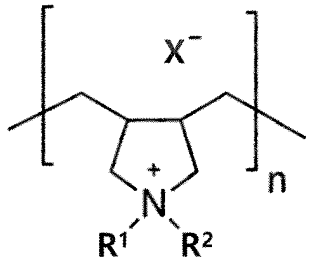

;

In Chemical Formula 1,

$R^1$ and $R^2$ are each independently an unsubstituted or substituted linear or branched $C_{1-10}$ alkyl group, an unsubstituted or substituted linear or branched $C_{2-10}$ alkenyl group, an unsubstituted or substituted linear or branched $C_{2-10}$ alkynyl group, or an unsubstituted or substituted $C_{5-10}$ cycloalkyl group,
$X^-$ is a halide ion, and
n is an integer of 10,000 to 10,000,000.

In an embodiment of the present disclosure, n may be an integer of about 10,000 to about 10,000,000, about 10,000 to about 9,000,000, about 10,000 to about 8,000,000, about 10,000 to about 7,000,000, about 10,000 to about 6,000,000, about 10,000 to about 5,000,000, about 10,000 to about 4,000,000, about 10,000 to about 3,000,000, about 10,000 to about 2,000,000, about 10,000 to about 1,000,000, about 10,000 to about 900,000, about 10,000 to about 800,000, about 10,000 to about 700,000, about 10,000 to about 600,000, about 10,000 to about 500,000, about 10,000 to about 400,000, about 10,000 to about 300,000, about 10,000 to about 200,000, or about 10,000 to about 100,000.

In an embodiment of the present disclosure, when $R^1$ and $R^2$ are substituted, they may be substituted with one or more substituents selected from halogen, hydroxyl, amino, carboxyl, cyano, nitro, formyl, and $C_{1-10}$ alkyl groups.

In an embodiment of the present disclosure, $X^-$ may be a fluoride ion, chloride ion, bromide ion, or iodide ion.

In an embodiment of the present disclosure, the quaternary ammonium salt may be selected from poly(diallyldimethylammonium) (PDDA) salt, poly(diallyldiethylammonium) salt, poly(diallyldipropylammonium) salt, poly(diallyldibutylammonium) salt, Poly(diallyldipentylammonium) salt, and poly(diallyldihexylammonium) salt.

In an embodiment of the present disclosure, a concentration of the graphene oxide may be about 1 ppb to about 10,000,000 ppb, but is not limited thereto.

In an embodiment of the present disclosure, the hair loss may include hormonal alopecia, alopecia areata, hereditary androgenetic alopecia, telogen effluvium, traumatic alopecia, trichotillomania, pressure alopecia, anagen effluvium, non-scarring alopecia, syphilitic alopecia, seborrheic alopecia, symptomatic alopecia, cicatricial alopecia, or congenital alopecia, but is not limited thereto.

In an embodiment of the present disclosure, the composition may adsorb male hormones. In an embodiment of the present disclosure, the male hormones may include testosterone, but are not limited thereto.

In an embodiment of the present disclosure, the hair loss may be hormonal alopecia (e.g., androgenetic alopecia).

In an embodiment of the present disclosure, the composition for preventing hair loss or promoting hair growth may prevent hair loss or promote hair growth by adsorbing testosterone and thereby inhibiting the production of dihydrotestosterone (DHT), which is formed from testosterone by the action of 5α-reductase. Alternatively, in an embodiment of the present disclosure, the composition may prevent hair loss or promote hair growth by adsorbing DHT.

In an embodiment of the present disclosure, the composition may promote the growth or proliferation of Human Follicle Dermal Papilla Cells, (PromoCell).

In an embodiment of the present disclosure, the composition may enhance the expression or activity of hair growth-promoting factor proteins in Human follicle dermal papilla cells.

In an embodiment of the present disclosure, the composition may enhance the expression or activity of one or more selected from VEGF (vascular endothelial growth factor), FGF7 (fibroblast growth factor 7), HGF (hepatocyte growth factor), IGF-1 (insulin-like growth factor-1), and β-catenin, but is not limited thereto.

In an embodiment of the present disclosure, the composition may enhance the expression or activity of Wnt/β-catenin activation in Human follicle dermal papilla cells.

In an embodiment of the present disclosure, the composition may suppress the expression of hair loss-inducing genes in Human follicle dermal papilla cells.

In an embodiment of the present disclosure, the composition may suppress the expression of one or more selected from AR, 5αR1, 5αR2, and DKK-1, but is not limited thereto.

In an embodiment of the present disclosure, the composition may be a cosmetic composition, a quasi-drug composition, or a pharmaceutical composition, but is not limited thereto.

In an embodiment of the present disclosure, the composition for preventing hair loss or promoting hair growth contains graphene oxide having an average diameter of about 100 nm or more. Compared to graphene oxide having an average diameter of less than 100 nm, the composition may exhibit superior effects in promoting the growth or proliferation of human follicle dermal papilla cells, in enhancing the expression or activity of hair growth-promoting factor proteins in human follicle dermal papilla cells, and in improving the expression or activity of Wnt/β-catenin. Also, the composition shows superior suppression of hair loss-inducing gene expression. Thus, the composition exhibits excellent effects in preventing hair loss or promoting hair growth.

In an embodiment of the present disclosure, the composition for preventing hair loss or promoting hair growth is administered or applied topically to the treatment site to exhibit effects in preventing hair loss or promoting hair growth. Thus, the composition may prevent side effects associated with conventional hair loss medications (for example, systemic side effects caused by inhibition of sex hormones).

A second aspect of the present disclosure provides a method for preventing hair loss or promoting hair growth, including administering to a subject the composition according to the first aspect.

Detailed descriptions of parts of the second aspect, which overlap with those of the first aspect, are omitted hereinafter, but the descriptions of the first aspect of the present disclosure may be identically applied to the second aspect of the present disclosure, even though they are omitted hereinafter.

In an embodiment of the present disclosure, the administration may include applying and/or injecting the composition to an application site of the subject, but is not limited thereto.

A third aspect of the present disclosure provides a cosmetic composition for preventing hair loss or promoting hair growth, including a graphene oxide, wherein an average diameter of the graphene oxide is about 100 nm or more.

Detailed descriptions of parts of the third aspect, which overlap with those of the first aspect and the second aspect, are omitted hereinafter, but the descriptions of the first aspect and the second aspect of the present disclosure may be identically applied to the third aspect of the present disclosure, even though they are omitted hereinafter.

In an embodiment of the present disclosure, the cosmetic composition may be in the form of hair tonic, hair conditioner, hair essence, hair lotion, hair nutrition lotion, hair shampoo, hair rinse, hair treatment, hair cream, hair nutrition cream, hair moisturizing cream, hair massage cream, hair wax, hair aerosol, hair pack, hair nutrition pack, hair soap, hair cleansing foam, hair oil, hair dryer agent, hair preservative, hair dye, hair waving agent, hair bleaching agent, hair gel, hair glaze, hair dressing agent, hair lacquer, hair moisturizer, hair mousse, hair spray, eyebrow growth agent, eyelash growth agent, eyelash nutrient, pet shampoo or rinse, skin toner, nutrition cream, massage cream, eye cream, eye essence, essence, cleansing cream, cleansing lotion, cleansing foam, cleansing water, pack, powder, body lotion, body cream, body essence, or body cleanser, but is not limited thereto.

In an embodiment of the present disclosure, the cosmetic composition may further contain other components in addition to the graphene oxide, depending on the intended use. For example, the cosmetic composition may further include components commonly contained in cosmetic compositions and/or functional additives. The components commonly contained in cosmetic compositions may include, but are not limited to, moisturizers, emollients, surfactants, UV absorbers, preservatives, bactericides, antioxidants, pH regulators, organic and inorganic pigments, fragrances, or cooling agents. The functional additives may include, but are not limited to, watersoluble vitamins, fat-soluble vitamins, polymeric peptides, or polymeric polysaccharides.

A fourth aspect of the present disclosure provides a quasi-drug composition for preventing hair loss or promoting hair growth, including a graphene oxide, wherein an average diameter of the graphene oxide is about 100 nm or more.

Detailed descriptions of parts of the fourth aspect, which overlap with those of the first aspect to the third aspect, are omitted hereinafter, but the descriptions of the first aspect to the third aspect of the present disclosure may be identically applied to the fourth aspect of the present disclosure, even though they are omitted hereinafter.

As generally defined, the term "quasi-drugs" refers to products used for treating or preventing diseases, or for alleviating symptoms, and the term "cosmetics" refer to products used for maintaining or promoting the health of skin or hair for the purpose of cleanliness or beautification of the human body.

The composition for preventing hair loss or promoting hair growth of the present disclosure may be classified as a quasi-drug composition when it contains graphene oxide at a sufficiently high content for treating, alleviating, or preventing hair loss, whereas it may be classified as a cosmetic composition when it contains graphene oxide at a lower content than that of the quasi-drug composition, thereby having mild pharmacological action and being used for maintaining or promoting hair health. Accordingly, the quasi-drug composition may have the same formulation as the cosmetic composition, but the graphene oxide content may be adjusted within a certain range to exhibit pharmacological action.

In an embodiment of the present disclosure, the quasi-drug composition may be in the form of hair tonic, hair conditioner, hair essence, hair lotion, hair nutrition lotion, hair shampoo, hair rinse, hair treatment, hair cream, hair nutrition

cream, hair moisturizing cream, hair massage cream, hair wax, hair aerosol, hair pack, hair nutrition pack, hair soap, hair cleansing foam, hair oil, hair dryer agent, hair preservative, hair dye, hair waving agent, hair bleaching agent, hair gel, hair glaze, hair dressing agent, hair lacquer, hair moisturizer, hair mousse, hair spray, eyebrow growth agent, eyelash growth agent, eyelash nutrient, pet shampoo or rinse, skin toner, nutrition cream, massage cream, eye cream, eye essence, essence, cleansing cream, cleansing lotion, cleansing foam, cleansing water, pack, powder, body lotion, body cream, body essence, or body cleanser, but is not limited thereto.

In an embodiment of the present disclosure, the quasi-drug composition may further contain other quasi-drug components in addition to the graphene oxide, depending on the intended use.

In an embodiment of the present disclosure, the graphene oxide content in the quasi-drug composition may be appropriately selected depending on the intended use (prevention, health maintenance, or therapeutic treatment).

A fifth aspect of the present disclosure provides a pharmaceutical composition for preventing hair loss or promoting hair growth, including a graphene oxide, wherein an average diameter of the graphene oxide is about 100 nm or more.

Detailed descriptions of parts of the fifth aspect, which overlap with those of the first aspect to the fourth aspect, are omitted hereinafter, but the descriptions of the first aspect to the fourth aspect of the present disclosure may be identically applied to the fifth aspect of the present disclosure, even though they are omitted hereinafter.

In an embodiment of the present disclosure, the pharmaceutical composition may be administered parenterally. The parenteral administration may include intramuscular, transdermal, or subcutaneous administration, but is not limited thereto.

In an embodiment of the present disclosure, the pharmaceutical composition may be administered by topical application to a diseased site or by injection of an injectable solution, but is not limited thereto.

In an embodiment of the present disclosure, the pharmaceutical composition may be formulated as a conventional pharmaceutical dosage form known in the relevant technical field. Specifically, the pharmaceutical composition may be formulated as an injectable preparation for intramuscular, transdermal, or subcutaneous administration, or as a skin external preparation for topical application.

In an embodiment of the present disclosure, the pharmaceutical composition may be in the form of injectable solution, cream, ointment, paste, gel, patch, jelly, serum, powder, aerosol spray, non-aerosol spray, foam, lotion, solution, or suspension, but is not limited thereto.

In an embodiment of the present disclosure, the pharmaceutical composition may further contain, for formulation into such dosage forms, sterilized aqueous solutions, nonaqueous solvents, suspensions, emulsions, lyophilized preparations, or external preparations. Specifically, when the dosage form is an injectable solution, it may further contain a stabilizer, a buffer, and/or water. When the dosage form is a paste, cream, or gel, it may further contain a carrier such as animal fibers, vegetable fibers, wax, paraffin, starch, tragacanth, cellulose derivatives, polyethylene glycol, silicone, bentonite, silica, talc, or zinc oxide. When the dosage form is a powder or spray, it may further contain a carrier such as lactose, talc, silica, aluminum hydroxide, calcium silicate, or polyamide powder. When the dosage form is a spray, it may further contain a propellant such as chlorofluorohydrocarbon, propane/butane, or dimethyl ether.

In an embodiment of the present disclosure, the pharmaceutical composition may further contain another medication suitable for treating hair loss.

Hereinafter, the present disclosure will be described in more detail with reference to Examples, but the present disclosure may not be limited thereto.

## MODE FOR CARRYING OUT THE INVENTION

[Examples]

Example 1: Preparation of Pharmaceutical Composition Containing Nanosized Graphene Oxide (GO)

1) Preparation of Graphene Oxide with Different Diameters

Graphene oxide was prepared by using Hummers' method. Graphite was sufficiently stirred in an $H_2SO_4$ solution at room temperature and then, $KMnO_4$ was added to the solution. Subsequently, $H_2O_2$ was further added to the solution to induce an oxidation reaction of graphite and thereby form graphene oxide. After several rounds of purification, graphene oxide was obtained. Then, the graphene oxide was dispersed in water to obtain an aqueous dispersion of graphene oxide. Graphene oxide fractionated into various diameter sizes was analyzed by laser diffraction (Mie scattering method). In the Mie scattering method, when light collides with a particle, it is scattered (diffracted), and a detector obtains different electrical signals depending on the angle and intensity of the scattered light. Based on Mie theory, the size distribution of samples (test products) can be obtained from the respective different electrical signals, and the size (average diameter) of each sample was adjusted accordingly.

2) Preparation of PDDA (poly(diallyldimethylammonium) salt)-Functionalized Graphene Oxide

Then, 30 mg of the dried graphene oxide, prepared as described in paragraph 1, was dispersed in 30 mL of ultrapure water (1 mg/mL, 0.1 wt%) and sonicated to prepare an aqueous dispersion of graphene oxide. Thereafter, a 35 wt% solution of poly(diallyldimethylammonium chloride) (Sigma-Aldrich) was added to the aqueous dispersion of graphene oxide and mixed with a magnetic stirrer at room temperature for 2 hours. The amount of the PDDA salt solution was adjusted to 30 μL to 600 μL. Subsequently, the transparent PDDA aqueous dispersion that had not reacted with the graphene oxide and remained separated was removed by means of centrifugation.

Test Example 1: In-vitro Evaluation

1) Test Method

(1) Cell culture

Human follicle dermal papilla cells (PromoCell) were cultured at 37°C in 5% $CO_2$ in Human Follicle Dermal Papilla Cell Growth Medium supplemented with fetal calf serum, bovine pituitary extract, basic fibroblast growth factor, and insulin.

(2) Preparation of Test Product

The test product was diluted in culture medium to 100,000 ppb and then serially diluted tenfold (1:9) with culture medium to obtain concentrations of 10 ppb, 100 ppb, and 1,000 ppb, which were subsequently applied to cells.

(3) Evaluation of Cell Proliferation Rate

The cultured cells were dispensed into a 96-well plate at $5\times10^3$ cells/well. After treatment with the prepared test product at each concentration, the cells were cultured for 24 hours, 48 hours, and 72 hours. Thereafter, WST substrate solution (CCK-8, Dojindo) was added to the medium and cultured at 37°C for 2 hours, and absorbance (OD) at 450 nm was measured using a microplate reader (VARIOSKAN LUX, Thermo Fisher Scientific). The measured OD values were used to calculate the cell proliferation rate according to Equation 1 below.
[Equation 1]

$$\text{Cell proliferation rate (\%)} = (\text{ODexp}-\text{ODblnk})/(\text{ODcon}-\text{ODblnk}) \times 100$$

- Blank (blnk): Medium containing WST substrate
- Control (con, (Negative) Control Group): Untreated test product
- Experiment (exp, Test Group): Treated test product

(4) Evaluation of Protein Production

The cultured cells were divided at $5\times10^4$ cells and dispensed into a 6-well plate. When the culture exceeded 80% confluency, the test product was added to each well at the indicated concentrations and cultured for 24 hours. Thereafter, the culture medium was collected and centrifuged at 2,000 ×g for 10 minutes, and only the supernatant excluding cell debris was collected to evaluate FGF7 and VEGF protein production.
Quantitation of VEGF protein used a Human VEGF ELISA kit (abcam), and quantitation of FGF-7 protein used a Human FGF-7 ELISA kit (abcam). The culture media were used neat, the test was conducted according to the enclosed instructions, OD values were measured with a microplate reader (VARIOSKAN LUX, Thermo Fisher Scientific), and protein concentrations were calculated by substituting the measured values into the regression equation obtained from the standard curve and multiplying by the dilution factor.

(5) Evaluation of Enzyme Activity

The cultured cells were divided at $5\times10^4$ cells and dispensed into a 6-well plate. When the culture exceeded 80% confluency, the cells were treated with $H_2O_2$ (400 μM) to induce oxidative stress, and the test product was applied at the indicated concentrations. Then, the cells were cultured again for 24 hours. Thereafter, the culture medium was collected

and centrifuged at 2,000 ×g for 10 minutes, and only the supernatant excluding cell debris was collected and used for the test.

SOD (superoxide dismutase) activity was measured using an SOD kit (BIOMAX). The culture media were used neat, the test was conducted according to the enclosed instructions, and OD values were measured with a microplate reader (VARIOSKAN LUX, Thermo Fisher Scientific). The measured OD values were used to calculate SOD activity according to the enclosed analysis method.

(6) Evaluation of Cellular Uptake

The cultured cells were divided at $1 \times 10^6$ cells and dispensed into a 100-mm dish. When the culture exceeded 80% confluency, the test product was applied at the indicated concentrations and the cells were cultured again for 24 hours. Thereafter, the culture medium was removed, and the cells were collected with a scraper and fixed in TEM (transmission electron microscopy)-dedicated fixer for 8 hours or more. After the cells were washed, they were fixed again with $OsO_4$ and dehydrated. After the cells were infiltrated with resin and embedded into the cells, ultrathin sections were prepared with an ultramicrotome to allow for electron beam penetration. Then, toluidine staining was performed to identify areas for TEM observation. After staining, grids were prepared, double-stained with uranyl/lead, and imaged by using TEM (JEM-1011, JEOL).

(7) Statistical Analysis

Statistical analysis was performed using IBM SPSS Statistics 25.0. Significance between the test and control groups was assessed at a hypothesized mean difference of 5% ($p<0.05$). After a normality test, significance was determined by an independent-samples t-test (parametric method) depending on whether normality was satisfied.

2) Result

(1) Evaluation of Cell Proliferation Rate Depending on Graphene Oxide Size at 10 ppb for 24 Hours

After 24 hours of cell culture at a graphene oxide concentration of 10 ppb, cell proliferation rates were evaluated depending on the size of graphene oxide (10 nm, 110 nm, 300 nm, 500 nm, 2,000 nm, and 7,500 nm) (Table 1 and **FIG. 1**). As shown in Table 1 and **FIG. 1**, at graphene oxide sizes of 110 nm, 300 nm, or 500 nm, the cell proliferation rates were about 128% or higher, which were significantly higher than those observed at 10 nm, 2,000 nm, or greater. Further, the highest cell proliferation rate was observed at a graphene oxide size of 110 nm, reaching 141.533%.

[Table 1]

| Size of graphene oxide (nm) | Cell proliferation rate (%) |
|---|---|
| 10 | 94.863 |
| 110 | 141.533 |
| 300 | 132.504 |
| 500 | 128.977 |
| 2000 | 80.901 |
| 7500 | 88.916 |

(2) Evaluation of Cell Proliferation Rate Depending on Graphene Oxide Size at 10 ppb for 72 Hours

After 72 hours of cell culture at a graphene oxide concentration of 10 ppb, cell proliferation rates were evaluated depending on the size of graphene oxide (10 nm, 110 nm, and 300 nm) (Table 2 and **FIG. 2**). As shown in Table 2 and **FIG. 2**, regardless of graphene oxide size, cell proliferation rates increased with longer culture time. Further, as observed under the 24 h/10 ppb conditions described above, the cell proliferation rates at graphene oxide sizes of 110 nm and 300 nm were significantly higher than the cell proliferation rate observed at 10 nm.

[Table 2]

| Size of graphene oxide (nm) | Cell proliferation rate (%) |
|---|---|
| 10 | 117.564 |
| 110 | 149.270 |
| 300 | 139.433 |

(3) Evaluation of Cell Proliferation Rate Depending on Concentration of 110-nm Graphene Oxide for 24 Hours

Under 24-h culture conditions, cell proliferation rates were evaluated depending on the concentration of 110-nm graphene oxide (1 ppb, 10 ppb, 100 ppb, 1,000 ppb, and 10,000 ppb)

(Table 3 and **FIG. 3**). As shown in Table 3 and **FIG. 3**, 110-nm graphene oxide showed high proliferation rates of about 129% or higher at concentrations of 1 ppb to 1,000 ppb, with the highest value of 141.533% observed at 10 ppb. **FIG. 4** shows optical microscopic images of cell morphology for 110-nm graphene oxide in the negative control and at concentrations of 1,000 ppb, 10,000 ppb, and 100,000 ppb under 24-h culture conditions. Morphological changes (arrows) were observed at concentrations of 10,000 ppb or higher.

[Table 3]

| Concentration (ppb) | Cell proliferation rate (%) |
|---|---|
| 1 | 129.898 |
| 10 | 141.533 |
| 100 | 137.975 |
| 1,000 | 133.340 |

(4) Evaluation of Hair Growth-Promoting Factor Protein Production

To compare 10-nm GO and 110-nm GO at a concentration of 10 ppb in human follicle dermal papilla cells, the production levels of hair growth-promoting factor protein (fibroblast growth factor (FGF7)) and vascular endothelial growth factor (VEGF) were evaluated (**FIG. 5** and **FIG. 6**, Table 4). The production levels of FGF7 and VEGF were significantly increased in the cells treated with 110-nm GO compared to the control group and 10-nm GO.

[Table 4]

| Size of graphene oxide (nm) | FGF7 Protein Production (pg/mL) | VEGF Protein Production (pg/mL) |
|---|---|---|
| Control group | 5.056±1.682 | 14.364±0.198 |
| 10 | 37.722±7.096** | 13.864±0.200 |
| 110 | 84.311±1.526*** | 36.241±0.365*** |
| **: p<0.01 by independent samples t-test vs (Negative) control ***: p<0.001 by independent samples t-test vs (Negative) control | | |

(5) Evaluation of Enzyme Activity

To compare 10-nm GO and 110-nm GO at a concentration of 10 ppb in human follicle dermal papilla cells, superoxide dismutase (SOD) enzyme activity was evaluated (**FIG. 7** and **Table 5**).

[Table 5]

| | SOD activity (%) |
|---|---|
| Control group (not treated with $H_2O_2$) | 304.903±4.765 |

(continued)

| | | SOD activity (%) |
|---|---|---|
| $H_2O_2$ | - | 251.993±10.685 |
| | GO 10 nm | 234.866±0.919 |
| | GO 110 nm | 290.245±5.108 |

As a result, a significant increase in SOD activity was observed in the cells treated with 110-nm GO compared to the $H_2O_2$-only treatment group, and SOD activity with 110-nm GO was higher than that with 10-nm GO.

Test Example 2: In vitro Evaluatio-2

1) Test Method

(1) Culture of Human Follicle Dermal Papilla Cell

Dermal papillae were isolated from the bulbs of excised hairs, transferred to culture dishes coated with collagen type I, and cultured in DMEM supplemented with penicillin (100 U/mL), streptomycin (100 μg/mL), and 20% heat-inactivated serum. The explants were left for one week, and once the dermal papillae adhered to the culture dish and cells began to outgrow, the medium was changed every three days. When the cells proliferated, they were collected using 0.25% trypsin/10 mM EDTA and maintained in DMEM supplemented with 10% heat-inactivated serum at 37°C in 5% $CO_2$. In the present test, passage-2 cells were treated with 10-nm graphene oxide or 110-nm graphene oxide under the indicated conditions for 24 hours, and RNA was then isolated.

(2) RNA Isolation and Real-Time PCR

RNA was isolated from the graphene oxide-treated cells by using a Qiagen RNeasy Mini Kit (Qiagen) according to the protocol. Then, 5 μg of cDNA was synthesized from the RNA by using a cDNA Synthesis Kit (Promega) containing ImProm-II™ reverse transcriptase and an oligo-dT primer. Thereafter, cDNA (1 μL) was amplified with each set of forward and reverse primers. Real-time PCR was performed by using a StepOnePlus Real-Time PCR System (Applied Biosystems, Foster City, CA). All the reactions used Power SYBR™ Green Premix (Applied Biosystems) with 50 ng of cDNA and 10 μM primers. Amplification was performed under the following conditions: 95°C for 10 minutes, followed by 40 cycles of 95°C for 15 seconds, and and 60°C for 60 seconds. Differences between the samples and the control group were calculated by using StepOnePlus Real-Time PCR Analysis Software (Applied Biosystems). The primer sequences used are shown in Table 6 below.

[Table 6]

| Gene | Oligonucleotide primers | |
|---|---|---|
| GAPDH | CGA CCA CTT TGT CAA GCT CA | AGG GGA GAT TCA GTG TGG TG |
| HGF | TCCACGGAAGAGGAGATGAGA | GGCCATATACCAGCTGGGAAA |
| IGF-1 | CCTCTCAAGAGCCACAAATGC | TCCAGCAGCCAAGATTCAGA |
| VEGF | AGGCCAGCACATAGGAGAGA | CGCGAGTCTGTGTTTTTGCA |
| FGF-7 | CAAGAGCACAATGCCCAAAA | CCTCAAGCCTTCATGACATTC |

2) Result

(1) Evaluation of Inhibition of Hair Loss-Inducing Gene Expression by Graphene Oxide

Dermal papilla (DP) cells were isolated from frontal and occipital hair follicles of male hair loss patients with high AR and 5αR1 expression, cultured, and subsequently used for testing. The DP cells were treated with 10-nm GO or 110-nm GO. After 24 hours, real-time PCR was used to evaluate the expression levels of hair loss-inducing genes (AR, 5αR1, and DKK-1). Referring to **FIG. 8A to FIG. 8C,** the expression levels of hair loss-inducing genes decreased in all the GO-treated groups compared to the control group. Further, the decrease in gene expression was greater with 110-nm GO than with 10-nm GO. In particular, inhibition of DKK-1 mRNA expression, known as a male hair loss-inducing factor, was

confirmed.

(2) Evaluation of Wnt/β-catenin Activation

The DP cells were transfected with the TOPFlash vector (TCF/LEF promoter) and treated with graphene oxide, and promoter activity was measured by luciferase assay. The Wnt/β-catenin pathway is an important signaling pathway related to hair growth. The Wnt/β-catenin signaling promotes the maintenance and activation of stem cells, which helps maintain hair health. Also, it stimulates cell division, which accelerates hair growth and contributes to new hair formation and regeneration. The pathway is activated when Wnt proteins bind to Frizzled receptors and LRP5/6 receptors on the cell surface. The activated Wnt signaling stabilizes β-catenin, which translocates into the nucleus and binds to the TCF/LEF promoter to promote the expression of a target gene.

Referring to **FIG. 9,** signaling activity increased in all the graphene oxide-treated groups compared to the control group. Thus, it can be seen that graphene oxide is involved in activating Wnt/β-catenin signaling.

(3) Confirmation of mRNA Expression of Cytokine Involved in Hair Growth

Various factors have been reported as hair growth-promoting factors. In particular, HGF (hepatocyte growth factor), IGF-1 (insulin-like growth factor-1), VEGF (vascular endothelial growth factor), and FGF7 (fibroblast growth factor 7) are known as representative hair growth factors.

The DP cells were treated with 10-nm GO or 110-nm GO at a concentration of 100 ppb. After 24 hours, RNA was isolated and reverse-transcribed with reverse transcriptase, and real-time PCR was performed with each primer to obtain results. Referring to **FIG. 10A** to **FIG. 10D,** the expression levels of HGF, IGF-1, VEGF, and FGF7 genes increased in both the 10-nm GO and 110-nm GO groups compared to the control group. Furthermore, the expression levels of all of these genes were higher in the 110-nm GO group than in the 10-nm GO group. This suggests that 110-nm GO may induce the expression of growth factors in DP cells more effectively than 10-nm GO and thereby promote hair growth.

Test Example 3: Ex-vivo Evaluation

1) Test Method

(1) Ex-vivo Human Hair Follicle Organ Culture

With the consent of patients undergoing hair transplantation at Kyungpook National University Hospital Hair Transplantation Center, occipital biopsy hair follicles were obtained. Hair follicle tissues were cut into follicular units and then cut straight below the sebaceous glands. The prepared follicles were cultured with 10-nm graphene oxide or 110-nm graphene oxide in Williams' E medium containing 2 mM L-glutamine, 10 ng/mL hydrocortisone, 10 μg/mL insulin, and penicillin/streptomycin. Then, 500 μL of a medium containing the substance was placed in a 24-well plate, and six follicles per condition were cultured for 6 days at 37°C in 5% $CO_2$. The medium containing the substance was replaced every three days without damaging the follicles. After 6 days of culture, images were taken and the length of outgrowth was measured. For each substance, tests were repeated three times to assess morphology and outgrowth length of follicles. On day 6, the hair follicle tissues were mounted on slides and imaged under a microscope (OLYMPUS, SZX9), and their lengths were then measured with iSolution Lite.

Reagents used - William's E (no phenol red, Gibco, A1217601), 2 mM L-glutamin (Gibco, 25030081), 10 ng/ml Hydrocortisone (Sigma, HO888), 10 μg/ml Insulin (Sigma, I2643), Penicillin/Streptomycin (Gibco, 10378016).

2) Result

(1) Evaluation of Hair Growth

Hair growth was evaluated after treatment with 10-nm and 110-nm PDDA-functionalized graphene oxide (PDDA-GO). Referring to **FIG. 11** and **FIG. 12,** hair growth was observed in the groups treated with 10-nm or 110-nm PDDA-GO at concentrations of 0.1 ppm and 1 ppm compared to the control group. Furthermore, , hair growth was greater with 110-nm PDDA-GO than with 10-nm PDDA-GO.

The above description of the present disclosure is provided for the purpose of illustration, and it would be understood by those skilled in the art that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner. Likewise, components described to be distributed can be implemented

in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A composition for preventing hair loss or promoting hair growth, comprising a graphene oxide, wherein an average diameter of the graphene oxide is 100 nm or more.

2. The composition of Claim 1,
wherein the average diameter of the graphene oxide is 100 nm to 500 nm.

3. The composition of Claim 1,
wherein the average diameter of the graphene oxide is 100 nm to 300 nm.

4. The composition of Claim 1,
wherein an average thickness of the graphene oxide is 100 nm to 2,000 nm.

5. The composition of Claim 1,
wherein the graphene oxide is functionalized with a quaternary ammonium salt.

6. The composition of Claim 1,
wherein the graphene oxide is functionalized with a quaternary ammonium salt represented by the following Chemical Formula 1:

[Chemical Formula 1]

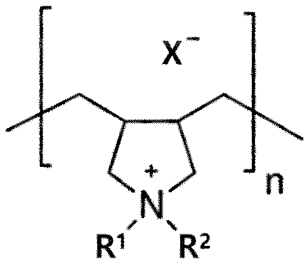

;

In Chemical Formula 1,

$R^1$ and $R^2$ are each independently an unsubstituted or substituted linear or branched $C_{1-10}$ alkyl group, an unsubstituted or substituted linear or branched $C_{2-10}$ alkenyl group, an unsubstituted or substituted linear or branched $C_{2-10}$ alkynyl group, or an unsubstituted or substituted $C_{5-10}$ cycloalkyl group,
$X^-$ is a halide ion, and
n is an integer of 10,000 to 10,000,000.

7. The composition of Claim 5,
wherein the quaternary ammonium salt is selected from poly(diallyldimethylammonium) (PDDA) salt, poly(diallyldiethylammonium) salt, poly(diallyldipropylammonium) salt, poly(diallyldibutylammonium) salt, Poly(diallyldipentylammonium) salt, and poly(diallyldihexylammonium) salt.

8. The composition of Claim 1,
wherein a concentration of the graphene oxide is 1 ppb to 10,000,000 ppb.

9. The composition of Claim 1,
wherein the composition promotes the growth or proliferation of Human Follicle Dermal Papilla Cells.

**10.** The composition of Claim 1,
wherein the composition enhances the expression or activity of one or more selected from VEGF (vascular endothelial growth factor), FGF7 (fibroblast growth factor 7), HGF (hepatocyte growth factor), IGF-1 (insulin-like growth factor-1), and β-catenin.

**11.** The composition of Claim 1,
wherein the composition suppresses the expression of one or more selected from AR, 5αR1, 5αR2, and DKK-1.

**12.** The composition of Claim 1,
wherein the hair loss is androgenetic alopecia.

**13.** The composition of Claim 1,
wherein the composition is a cosmetic composition, a quasi-drug composition, or a pharmaceutical composition.

**14.** A method for preventing hair loss or promoting hair growth, comprising
administering to a subject the composition according to Claim 1.

**15.** The method of Claim 14,
wherein the administration includes applying and/or injecting the composition to an application site of the subject.

**16.** A cosmetic composition for preventing hair loss or promoting hair growth, comprising a graphene oxide, wherein an average diameter of the graphene oxide is 100 nm or more.

**17.** A quasi-drug composition for preventing hair loss or promoting hair growth, comprising a graphene oxide, wherein an average diameter of the graphene oxide is 100 nm or more.

**18.** A pharmaceutical composition for preventing hair loss or promoting hair growth, comprising a graphene oxide, wherein an average diameter of the graphene oxide is 100 nm or more.

[FIG. 1]

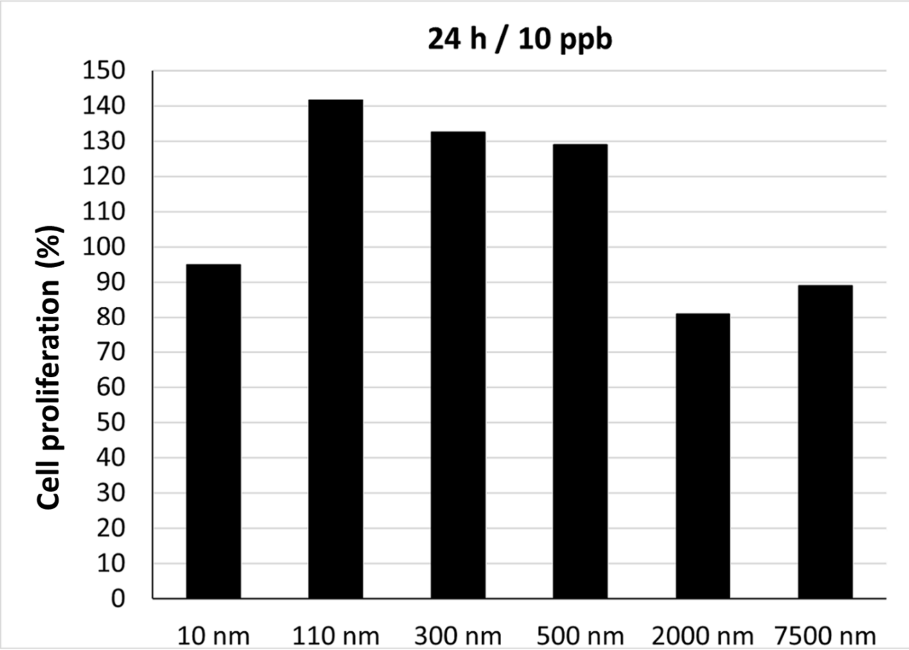
24 h / 10 ppb
Cell proliferation (%)
150
140
130
120
110
100
90
80
70
60
50
40
30
20
10
0
10 nm
110 nm
300 nm
500 nm
2000 nm
7500 nm

[FIG. 2]

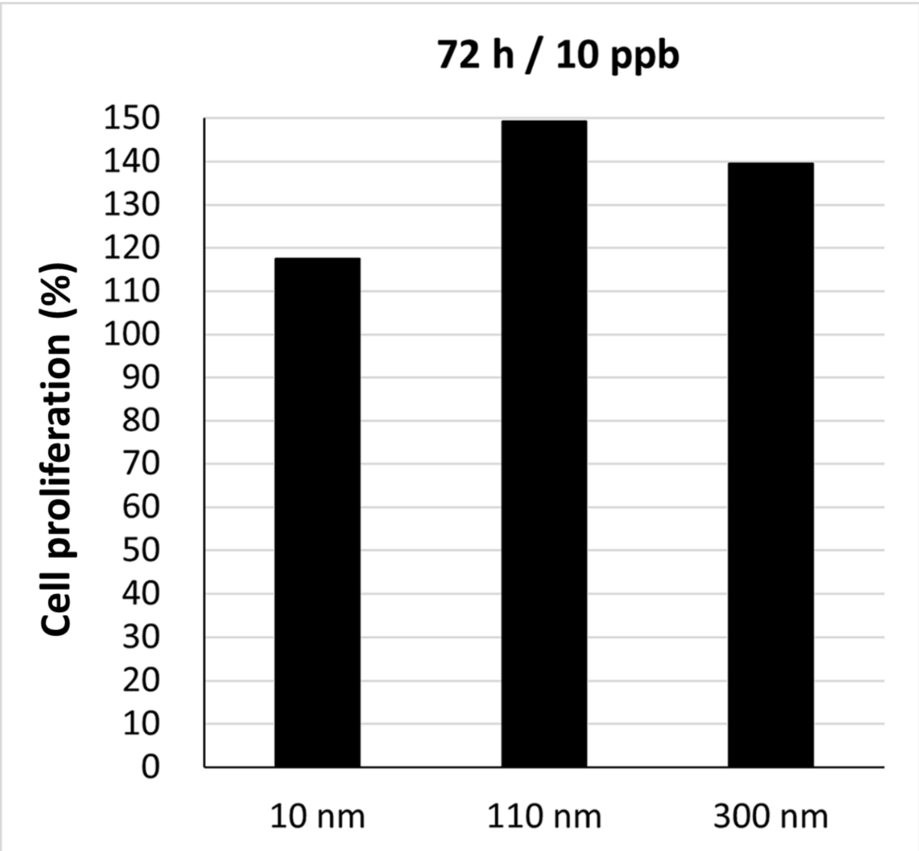
72 h / 10 ppb
Cell proliferation (%)
150
140
130
120
110
100
90
80
70
60
50
40
30
20
10
0
10 nm
110 nm
300 nm

[FIG. 3]

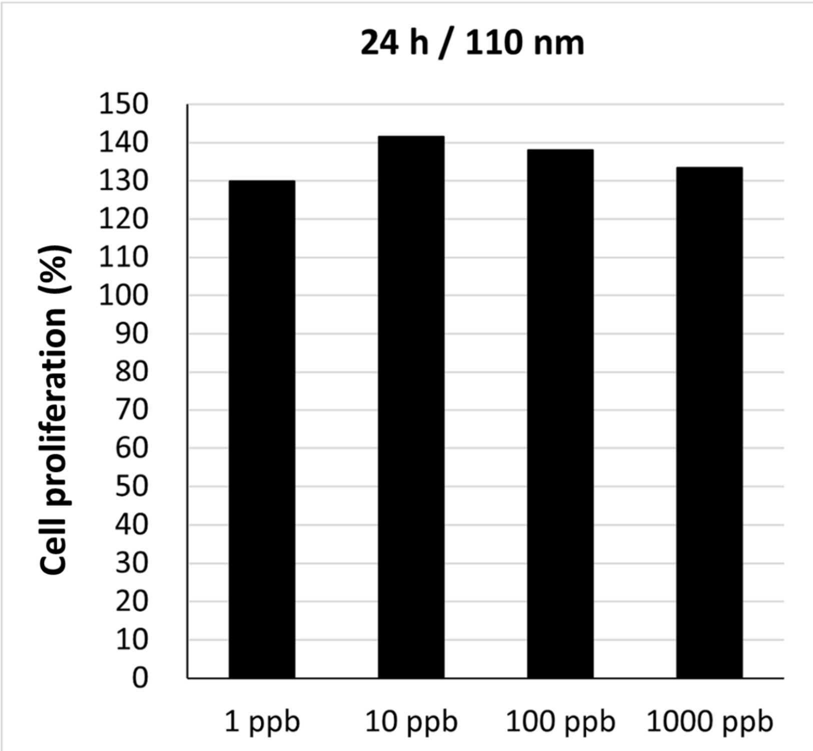
24 h / 110 nm
Cell proliferation (%)
150
140
130
120
110
100
90
80
70
60
50
40
30
20
10
0
1 ppb
10 ppb
100 ppb
1000 ppb

[FIG. 4]

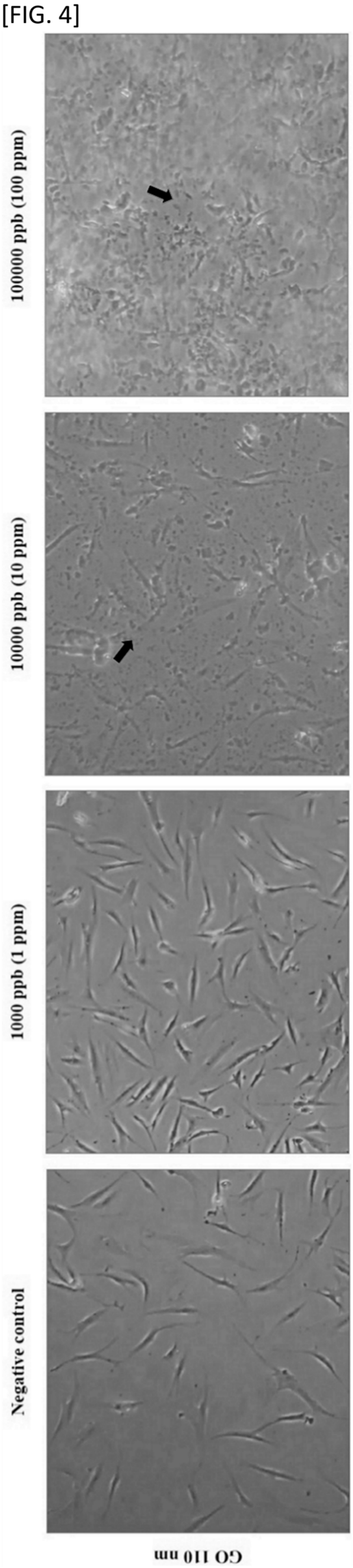
100000 ppb (100 ppm)
10000 ppb (10 ppm)
1000 ppb (1 ppm)
Negative control
GO 110 nm

[FIG. 5]

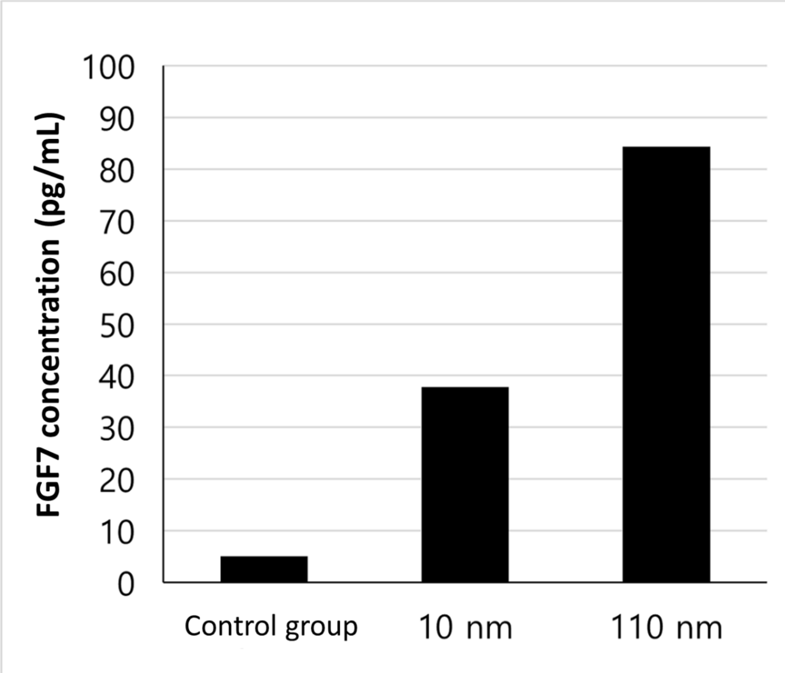
FGF7 concentration (pg/mL)
100
90
80
70
60
50
40
30
20
10
0
Control group
10 nm
110 nm

[FIG. 6]

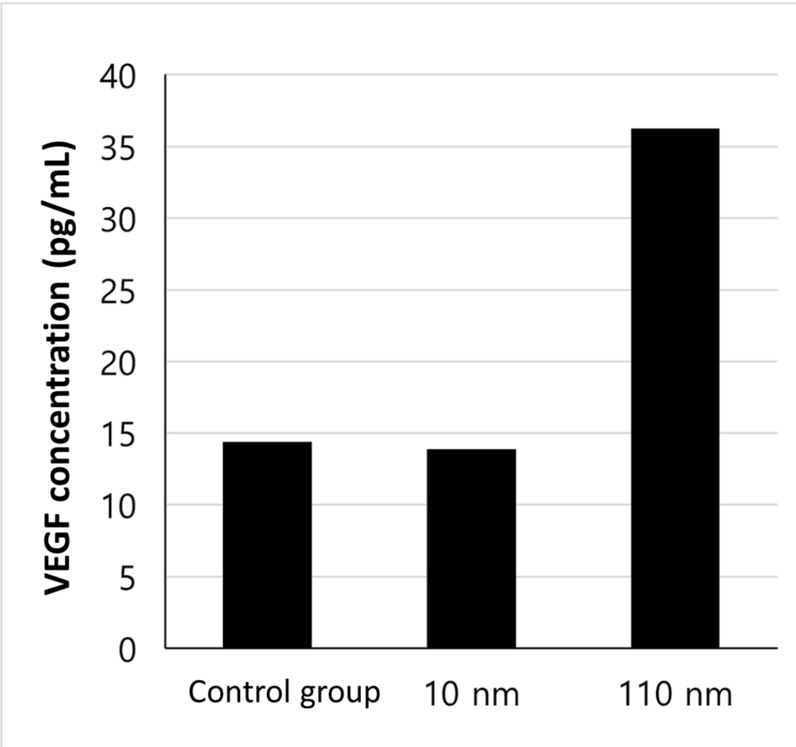
VEGF concentration (pg/mL)
40
35
30
25
20
15
10
5
0
Control group
10 nm
110 nm

[FIG. 7]

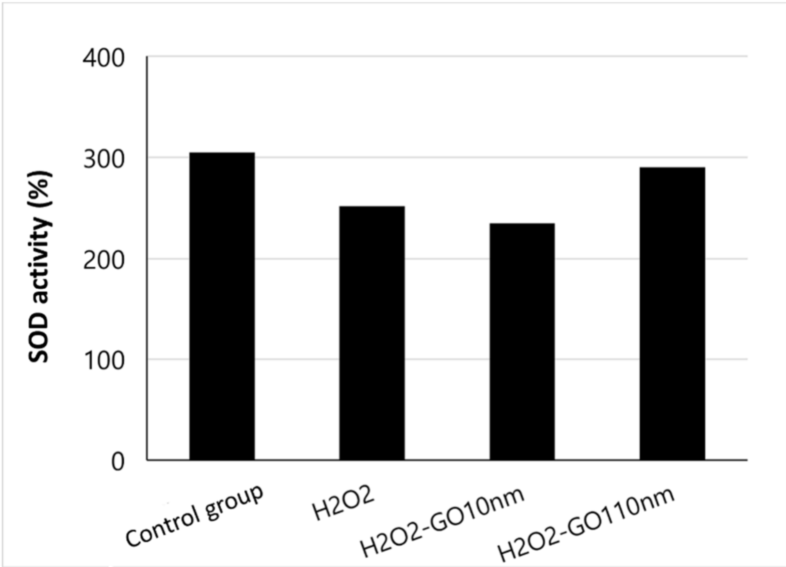


[FIG. 8A]

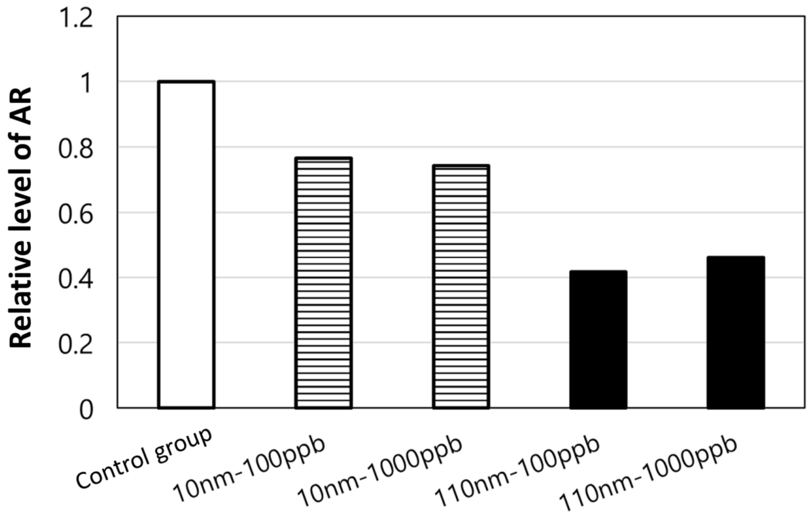


[FIG. 8B]

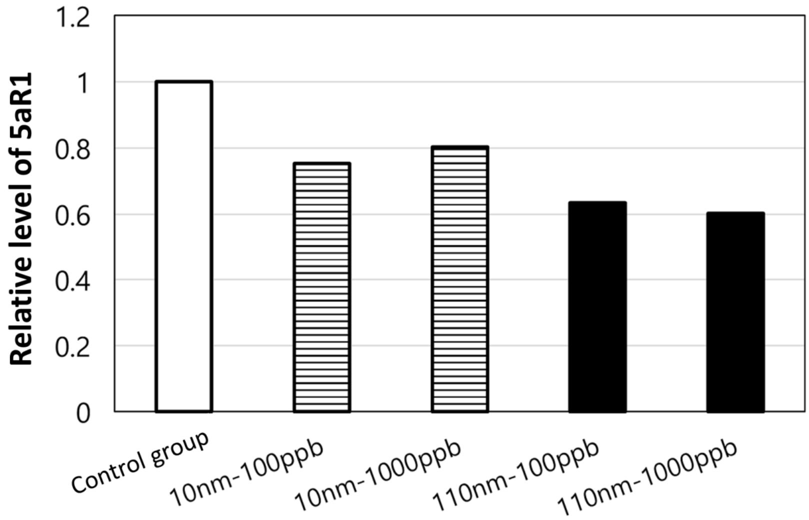


[FIG. 8C]

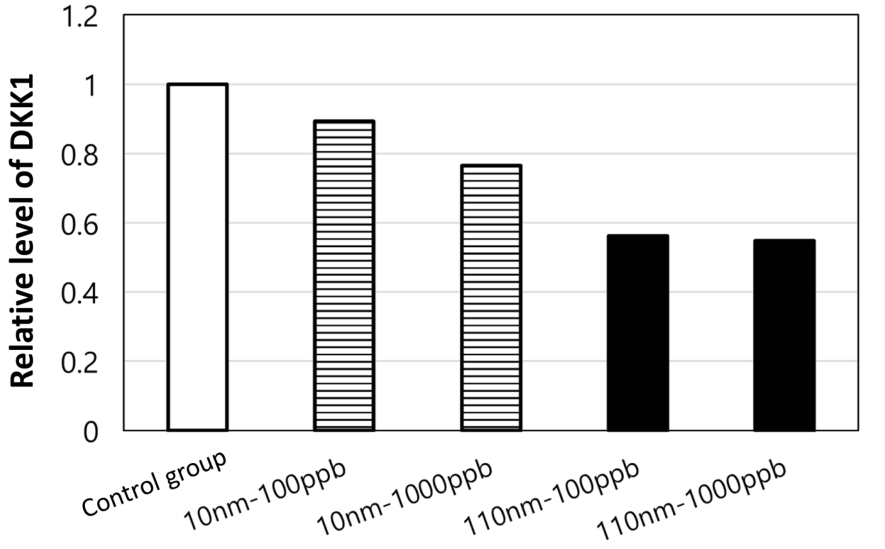


[FIG. 9]

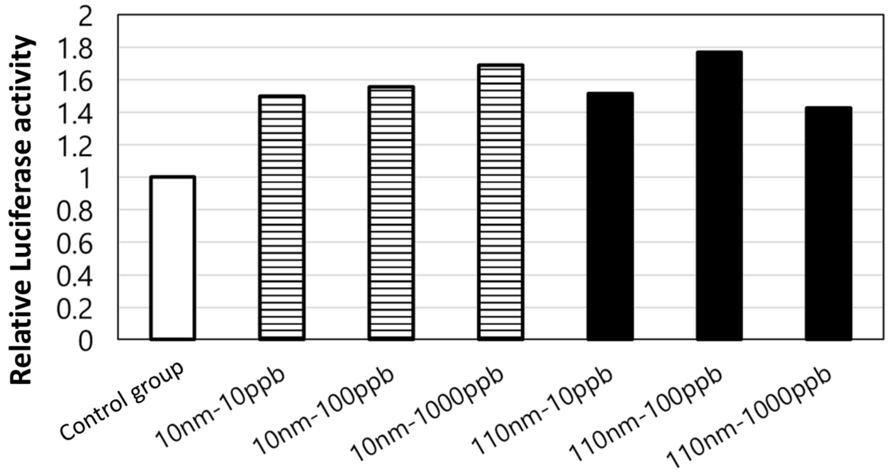


[FIG. 10A]

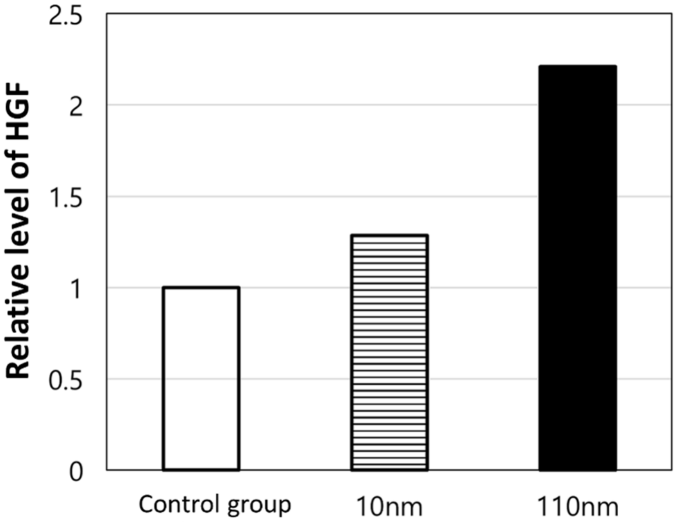


[FIG. 10B]

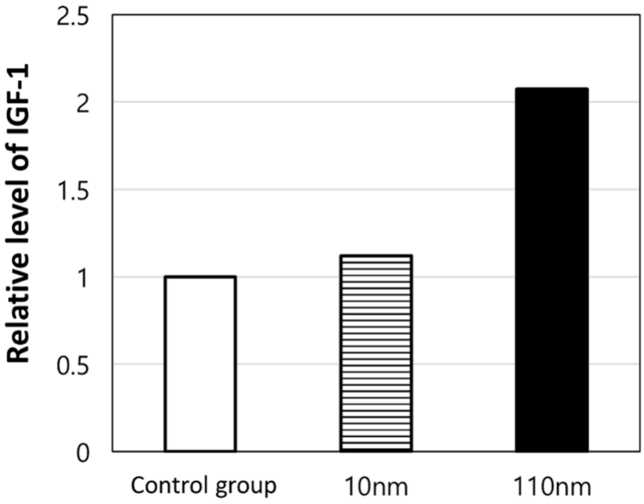


[FIG. 10C]

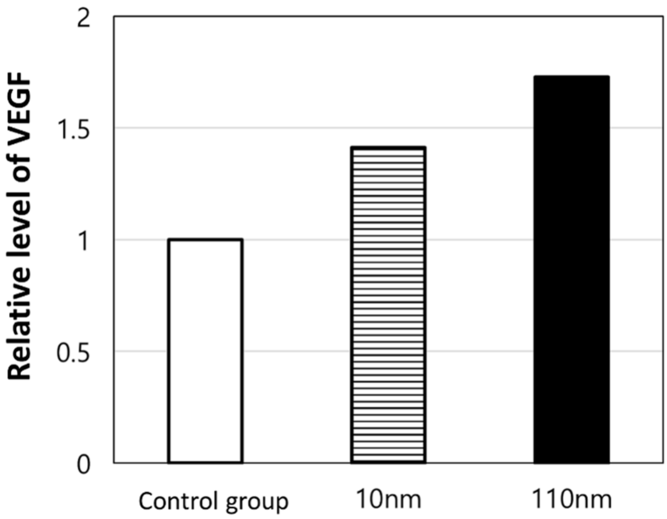


[FIG. 10D]

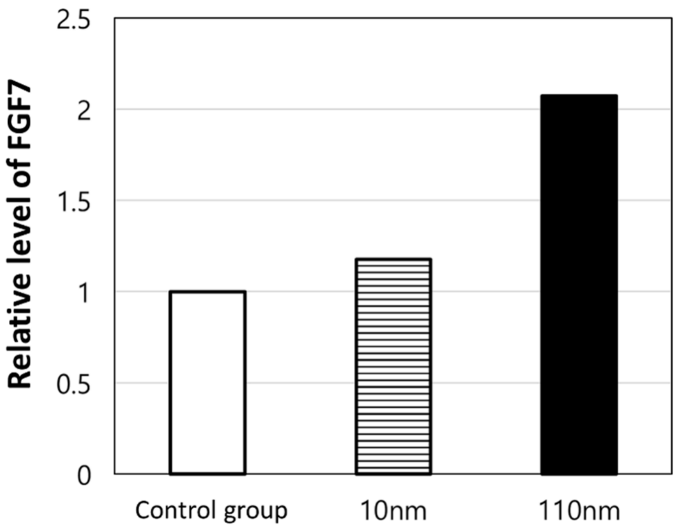


[FIG. 11]

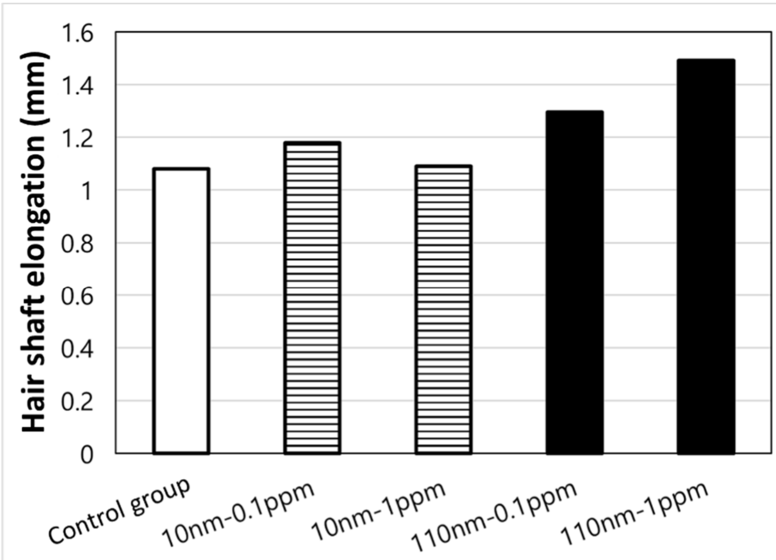
Hair shaft elongation (mm)
1.6
1.4
1.2
1
0.8
0.6
0.4
0.2
0
Control group
10nm-0.1ppm
10nm-1ppm
110nm-0.1ppm
110nm-1ppm

[FIG. 12]

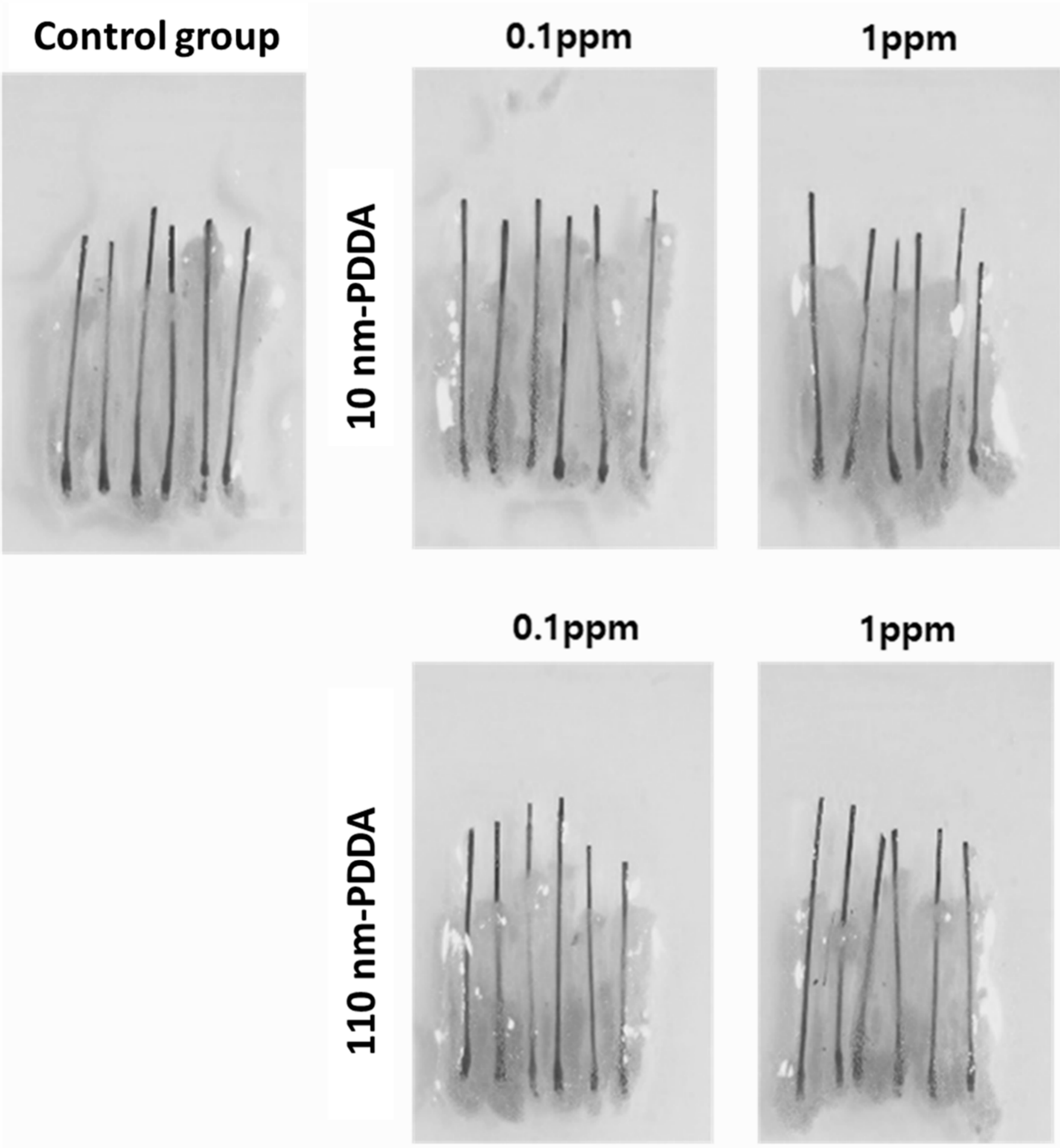

## INTERNATIONAL SEARCH REPORT

International application No.
**PCT/KR2024/010403**

**A. CLASSIFICATION OF SUBJECT MATTER**

**A61K 33/44**(2006.01)i; **A61P 17/14**(2006.01)i; **A61P 43/00**(2006.01)i

According to International Patent Classification (IPC) or to both national classification and IPC

**B. FIELDS SEARCHED**

Minimum documentation searched (classification system followed by classification symbols)

A61K 33/44(2006.01); A23K 20/20(2016.01); A23L 33/10(2016.01); A61K 8/19(2006.01); A61K 8/34(2006.01); A61K 8/44(2006.01); A61K 9/51(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 산화 그래핀(graphene oxide), 탈모(alopecia), 4차 암모늄화합물(quaternary ammonium compound)

**C. DOCUMENTS CONSIDERED TO BE RELEVANT**

| Category* | Citation of document, with indication, where appropriate, of the relevant passages | Relevant to claim No. |
|---|---|---|
| X<br>A | KR 10-2019-0117892 A (LEE, Jin Sun) 17 October 2019 (2019-10-17)<br>See claim 1; and paragraph [0018]. | 1-4,8,13,16-18<br>5-7,9-12 |
| X | KR 10-2019-0060491 A (SEOUL NATIONAL UNIVERSITY R&DB FOUNDATION et al.) 03 June 2019 (2019-06-03)<br>See claims 1 and 5; and paragraph [0016]. | 1-4,8,13,16-18 |
| A | KR 10-2019-0089306 A (LEE, Jin Sun) 31 July 2019 (2019-07-31)<br>See entire document. | 1-13,16-18 |
| A | WO 2019-103541 A1 (SEOUL NATIONAL UNIVERSITY R&DB FOUNDATION et al.) 31 May 2019 (2019-05-31)<br>See entire document. | 1-13,16-18 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 November 2024** | **04 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/010403**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14,15**
because they relate to subject matter not required to be searched by this Authority, namely:

Claims 14 and 15 pertain to a method for treatment of the human body ((PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/010403**

| Patent document cited in search report | Publication date (day/month/year) | Patent family member(s) | Publication date (day/month/year) |
|---|---|---|---|
| KR 10-2019-0117892 A | 17 October 2019 | KR 10-2052262 B1 | 08 January 2020 |
| KR 10-2019-0060491 A | 03 June 2019 | CN 111542329 A | 14 August 2020 |
| | | CN 111542329 B | 13 December 2022 |
| | | EP 3714891 A1 | 30 September 2020 |
| | | EP 3714891 B1 | 14 February 2024 |
| | | JP 2021-503512 A | 12 February 2021 |
| | | JP 6994726 B2 | 04 February 2022 |
| | | KR 10-2031712 B1 | 14 October 2019 |
| | | US 11786549 B2 | 17 October 2023 |
| | | US 2020-0360429 A1 | 19 November 2020 |
| | | WO 2019-103541 A1 | 31 May 2019 |
| KR 10-2019-0089306 A | 31 July 2019 | None | |
| WO 2019-103541 A1 | 31 May 2019 | CN 111542329 A | 14 August 2020 |
| | | CN 111542329 B | 13 December 2022 |
| | | EP 3714891 A1 | 30 September 2020 |
| | | EP 3714891 B1 | 14 February 2024 |
| | | JP 2021-503512 A | 12 February 2021 |
| | | JP 6994726 B2 | 04 February 2022 |
| | | KR 10-2031706 B1 | 14 October 2019 |
| | | KR 10-2031712 B1 | 14 October 2019 |
| | | US 11786549 B2 | 17 October 2023 |
| | | US 2020-0360429 A1 | 19 November 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 102506673